# EUROPEAN PATENT SPECIFICATION

(11) **EP 4 236 752 B1**
(45) Date of publication and mention of the grant of the patent: **17.12.2025**
(21) Application number: 21916312.8
(22) Date of filing: 27.12.2021
(51) Int. Cl.: A61B 1/04, A61B 1/05, A61B 1/07

(54) **SYSTEM FOR MULTI-FUNCTIONAL MINIATURIZED ENDOSCOPIC IMAGING AND ILLUMINATION**
SYSTEM ZUR MULTIFUNKTIONALEN MINIATURISIERTEN ENDOSKOPISCHEN BILDGEBUNG UND BELEUCHTUNG
SYSTÈME POUR L'IMAGERIE ET L'ÉCLAIRAGE ENDOSCOPIQUES MULTIFONCTION MINIATURISÉS

(30) Priority: 28.12.2020 US 202063130856 P
(43) Date of publication of application: 06.09.2023
(73) Proprietor: Leadoptik Inc., San Jose, CA 95131 (US)
(72) Inventor: KHORASANINEJAD, Mohammadreza, Milpitas, CA 95035 (US)
(74) Representative: Boult Wade Tennant LLP
(86) International application number: PCT/US2021/065237
(87) International publication number: WO 2022/146934

(56) References cited:
- DE-A1- 102015 102 595
- US-A1- 2007 191 682
- US-A1- 2008 304 123
- US-A1- 2013 012 783
- US-A1- 2016 227 990
- US-A1- 2016 265 747
- US-A1- 2017 322 079
- US-A1- 2018 011 249
- US-A1- 2018 017 806
- US-A1- 2018 140 172
- US-A1- 2018 140 172
- US-A1- 2018 303 326
- US-A1- 2019 212 761
- US-A1- 2020 150 347
- US-A1- 2020 318 810
- US-B2- 10 512 518
- BASSET G ET AL: "Extra flat, flexible and disposable endoscope for lateral imaging", PROGRESS IN BIOMEDICAL OPTICS AND IMAGING, SPIE - INTERNATIONAL SOCIETY FOR OPTICAL ENGINEERING, BELLINGHAM, WA, US, vol. 9691, 8 March 2016 (2016-03-08), pages 96910H - 96910H, XP060064113, ISSN: 1605-7422, ISBN: 978-1-5106-0027-0, DOI: 10.1117/12.2209627

## Description

### FIELD OF THE INVENTION

The present invention relates generally to miniaturized optical imaging and illuminating systems, and apparatus, for example as may be used to realize miniaturized medical imaging based on the optical coherence tomography technique.

### BACKGROUND OF THE INVENTION

Accurate diagnosis and treatment of diseases in internal organs such as the pulmonary airways, the coronary arteries, and the gastrointestinal tract are difficult due to the inaccessibility of lesions. This is the main drive behind the miniaturization of optical imaging and illuminating (for therapeutic purposes) systems. One of the commonly used imaging systems is the endoscopic optical coherence tomography (OCT) catheter. In a typical endoscopic catheter, optical power is delivered via an optical fiber to the distal end of the catheter and then is redirected and focused into the tissue via several cascaded optical components. Two common approaches of redirecting and focusing the light are based on (i) gradient-index (GRIN) lenses and prisms and (ii) angle polished ball lenses. In the former, the GRIN lens focuses the light and then the prism redirects the light toward the tissue (in the radial direction, relative to fiber) where one needs to perform imaging and/or light illumination. The latter can be seen as a prism and a lens integrated into one device, where an angle polished facet section redirects (often by 90 degrees) the light coming from the fiber toward the lens, and the lens focuses the light into a tissue. In the case of imaging, scattered light from the tissue is collected by the same lens and re-directed toward the fiber via angle polished facet. Then the fiber delivers this light to the post-processing system (often to an interferometric arm and detectors) for processing and forming images. The endoscopic catheter (including but not limited to fiber connector, sheath, torque coil, fiber, ferrule, and other optical components attached to it) is moved back and forth and rotated about its axis (e.g., about a longitudinal axis which runs the length of the fiber) to reconstruct a 3D image of the scene (e.g., tissue).

In US 2008/304123 A1, an ultra high speed miniature two dimensional electrooptic image acquisition system uses prisms of varying geometries to control the amount of horizontal deflection and a Bragg grating to control the amount of vertical deflection. A collimating lens array and a Gaussian profile Bragg grating help confine the beam diameter of the deflected light beam. A separate prism further bends light into the vertical direction. A spherical lens focuses light onto a photodetector array for display.

In US 2018/0140172 A1, a multi-channel optical imaging and stimulation system includes a light source to deliver light beams into a light guide. Different light beams are coupled into different spatial modes supported by the light guide. The light guide includes multiple segments, each of which defines a window to couple a specified group of spatial modes out of the light guide to illuminate or stimulate a target. Light reflected, scattered, or emitted by the target is also collected by the windows in the light guide. The light collected by different windows is detected by different pixels of a detector, thereby creating a correspondence between the pixel location and the spatial location of site at which the light is collected. An image of the target is then reconstructed based on this correspondence.

An article entitled "Extra flat, flexible and disposable endoscope for lateral imaging" by G. Basset et al. (PROGRESS IN BIOMEDICAL OPTICS AND IMAGING, SPIE -INTERNATIONAL SOCIETY FOR OPTICAL ENGINEERING, BELLINGHAM, WA, US, vol. 9691, 8 March 2016 (2016-03-08), pages 96910H-96910H) describes a disposable endoscope based on extra flat flexible polymer slabs used as multimode waveguides.

### SUMMARY

The present invention is defined by the appended claims.

According to some embodiments, an apparatus for dynamically controlling propagation directions and shape of light (e.g., focusing, expanding/condensing the beam width, coupling in and out of waveguide), sorting light based on its properties (e.g., polarization and/or wavelength) is disclosed.

Some embodiments include a light source, optical fiber, photonic integrated circuit (PIC) components (e.g., ridge waveguide, tapered waveguide, ring resonator, Mach-Zehnder, array waveguide grating, input/output grating coupler), and the refractive and diffractive optical components (e.g., diffractive lens, gratings, metasurface-based lenses, refractive lenses, diffractive grating, surface relief grating, substate, liquid crystal) to control, shape, sort, and guide the light toward a desired direction and ultimately focus it into an object for imaging and/or illumination. Further, embodiments may include at least one optical source, at least one sensor, and at least one control module. The control module may control, tune, and adjust the functionality of each component depending on feedback from the sensor or user. The functionality of some components can be dynamically changed by applying an electric voltage and/or current or changing the properties of impinging light (e.g., polarization, wavelength). Further, the polarization state of light may be linear, circular, elliptical, random, unpolarized, or any combination of them.

According to some embodiments, methods disclosed herein may include a step of receiving, using a communication device or sensor, feedback data from at least one sensor, or image processing software. Using this feedback, the control module adjusts the functionality of various components, changes wavelength, polarization, or other properties of the light.

### BRIEF DESCRIPTION OF THE DRAWINGS

The accompanying drawings presented in this disclosure partially constitute the disclosure and illustrate different embodiments of the inventions. Furthermore, the drawings may contain captions and/or text that may explain certain embodiments of the present disclosure. These text and captions are included for non-limiting, explanatory/illustrative purposes of certain embodiments described in the present disclosure.
**FIGs. 1A-C** are schematics of two embodiments of miniaturized endoscopic fiber-based catheters.
**FIGs. 2A-C** show multi-spectral and multi-focal imaging and illuminating embodiments using Photonic Integrated Circuit (PIC)-based lenses.
**FIGs. 3A-B** show a multi-spectral and multi-focal imaging embodiment using Photonic Integrated Circuit (PIC)-based gratings.
**FIGs. 4A-C** show multi-spectral and multi-focal imaging embodiments based on cascaded gratings.
**FIGs. 5A-C** show three different embodiments of reconfigurable multi-focal imaging and illuminating systems.
**FIGs. 6A-B** show an embodiment of a miniaturized polarization-resolved imaging and illuminating system.
**FIG. 7** shows an embodiment of a fiber-based catheter imaging and illuminating system with an extended depth of focus.
**FIG. 8** shows a compact form factor embodiment to expand the beam's width.
**FIG. 9** is an exploded view of an embodiment of an optical imaging and illuminating system.
**FIG. 10** is a block diagram of different modules for implementing the methods disclosed herein, in accordance with some embodiments.

### DETAILED DESCRIPTION

Embodiments of the present invention are described more fully hereinafter with reference to the accompanying drawings, however, alternative configurations and embodiments are also possible without departing from the scope of the present application. Thus, the present application should not be construed as limited to the embodiments set forth herein. Rather, the illustrated and described embodiments are provided as examples to convey the scope of the invention as defined in the appended claims to those skilled in the art. In the drawings, the size and relative sizes of layers and regions may be exaggerated for clarity.

It will be understood that, although the terms first, second, third, etc. may be used herein to describe various elements, components, regions, layers, and/or sections, these elements, components, regions, layers, and/or sections should not be limited by these terms. These terms are used to distinguish one element, component, region, layer, or section from another region, layer, or section. Thus, a first element, component, region, layer, or section discussed below could be termed a second element, component, region, layer, or section without departing from the teachings of the present disclosure.

As used herein, the singular forms "a," "an," and "the" are intended to include the plural forms as well, unless the context clearly indicates otherwise. It will be further understood that the terms "comprises" and/or "compromising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and/or the present specification and will not be interpreted in an idealized or overly formal sense unless expressly so defined herein.

The optical illumination and imaging systems described above have various drawbacks. For example, systems that rely on GRIN and ball lenses suffer from significant optical aberration, including spherical aberration and astigmatism, which degrade the imaging resolution. Although one can mitigate these aberrations by cascading several lenses similar to microscope objective lenses, the large size and high cost of systems with multiple lenses render this approach expensive and impractical.

Besides the low imaging resolution, refractive-based lenses have limited functionality. They cannot perform polarization-resolved imaging or multispectral imaging, and their focal lengths are fixed (i.e., cannot be adjusted or changed). Furthermore, these lenses should be cascaded with other bulky optical components such as prisms to do imaging in the radial direction, which hinders further miniaturization of the imaging system. Because most of the fiber-based endoscope designs are based on the finite/finite conjugate design (point to point focusing and imaging, fiber core to focal spot, and vice versa) the optical path error (e.g., due to fabrication tolerances) between the fiber and lens not only can cause aberration and reduce the resolving power but also can change the effective focal length of the imaging system. Due to its solid nature, using a prism makes it very difficult to place any other component between the fiber and the lens; thus limiting the functionality of the whole system (for example one cannot control or sort the light, which is transmitted between fiber and lens via a prism, based on its polarization and/or wavelength). Also, both refractive lenses and prisms are passive optical components, which prevents the tunability of the optical system. In this disclosure, several systems and methods are described which address these problems and shortcomings.

The present disclosure describes devices, apparatuses, and systems to facilitate light control for imaging and illumination purposes in a compact and miniaturized form factor. Further, the present disclosure describes various methods to enable multi-focal imaging, multi-spectral imaging, and polarization-resolved imaging. Further, the present disclosure relates generally to small form factor optical systems to focus light into tissue/organs for imaging and/or illumination via an optical fiber and stack of miniaturized optical components and devices.

In the present disclosure, the term input aperture may refer to an aperture where light enters the optical imaging and illuminating system. Input aperture may be the facet of a single-mode or multimode fiber. Input aperture may be the facet of a laser. Input aperture may have different sizes, smaller or larger than 5 microns, smaller or larger than 10 microns, smaller or larger than 50 microns. Input aperture may have a different angle relative to the axial direction, it may be smaller or larger than 98 degrees, it may be smaller or larger than 90 degrees, it can be smaller or larger than 82 degrees.

In the present disclosure, the term focusing component may refer to any component that may focus the light or converge the light. Focusing component also may refer to a diffractive lens, refractive lens, metasurface-based lens, and/or a lens based on a photonic integrated circuit. The focusing component may compromise at least one lens, at least one lens stacked with a polarizer, at least one lens stacked with liquid crystal or waveplate. The focusing component may focus the incident light toward an oblique direction.

In the present disclosure, the term exit window may refer to the area where the light will exit from the optical imaging and illuminating system toward the object (to be illuminated or be imaged). Exit window may be a lens, and/or a stack of the lens and other components. Other components may be polarization components, liquid crystal, other lenses (e.g., diffractive, refractive, or metasurface lenses). There might be a plastic or glass enclosure or tube between the exit window and the object (e.g., tissue) to be illuminated or to be imaged.

In the present disclosure, the term optical routing structure may refer to any structure which may change the direction and/or angle of light. Optical routing structure may change the direction of light propagation utilizing diffraction, reflection, and/or refraction effects or any combination of these effects. An optical routing structure may be comprised of at least one diffractive optical component (e.g. grating, diffractive lens, metasurface-based lens, hologram), or at least one refractive optical component (e.g. mirror, partial mirror, dielectric mirror) or any other components such as polarizers wave-plates, liquid crystals. Optical routing structure may change the direction of light propagation depending on light polarization, angle, wavelength, or any combination of light properties.

In the present disclosure, the term transverse direction may refer to a direction not aligned with the length of the fiber (i.e., X- or axial direction). The transverse direction may refer to a direction aligned with the radius of the fiber (i.e., YZ plane). The transverse direction may refer to a direction in the XY plane or the XZ plane and has an angle with X-direction. The transverse direction may have a non-zero angle with an X-direction. The non-zero angle may be smaller or larger than 90 degrees, smaller or larger than 80 degrees, smaller or larger than 70 degrees.

In the present disclosure, the term oblique direction may refer to a direction different from the axial direction (i.e., X-direction). The angle difference between oblique direction and axial direction may be larger or smaller than 5 degrees, larger or smaller than 10 degrees, larger or smaller than 20 degrees, larger or smaller than 45 degrees, larger or smaller than 60 degrees larger, and/or smaller than 90 degrees.

In the present disclosure, the grating may refer to diffractive grating, subwavelength grating, surface relief grating, blazed grating, binary grating, metasurface-based grating, single-mode grating, multimode grating, polarization-dependent grating, and polarization-independent grating. The grating may operate in reflection and /or transmission mode.

In the present disclosure, diffractive components (e.g., grating, lens) are arrays of subwavelength scatterer, resonator, and/or nanostructures (building blocks). These building blocks can individually or collectively control the basic properties of light such as phase, amplitude, polarization, spatial and temporal profile, the direction of propagation, or combinations of these properties at the same time. Diffractive components' building blocks may be made of semiconductors (e.g., amorphous silicon, polycrystalline silicon, silicon carbide, gallium nitride, gallium phosphide), crystals (e.g., silicon, lithium niobate, diamond), dielectrics (e.g., silicon dioxide, silicon nitride, aluminum oxide, hafnium oxide, titanium dioxide, indium oxide), polymers (e.g., photoresist, PMMA), metals (e.g., silver, aluminum, gold), two-dimensional (2D) materials (e.g., graphene, boron nitride), phase change materials (e.g., chalcogenide, vanadium dioxide) or any mixtures or alloys thereof.

In the present disclosure, metasurfaces are advanced forms of diffractive components and may refer to meta-gratings (gratings based on metasurface designs), meta-lenses (metasurface-based lens), and meta-hologram (holograms based on metasurface designs). These metasurfaces may be fabricated using various approaches such as optical lithography, deep-ultraviolet lithography, electron beam lithography, nanoimprint lithography, reactive ion etching, electron beam deposition, sputtering, plasma-enhanced deposition, atomic layer deposition, and combinations of the aforementioned processes.

Throughout the present disclosure, dynamic components or design or in general the adjective "dynamic" as used herein may refer to components or designs having function, performance, and properties can be adjusted at a different time through changing the properties of light (e.g., polarization, wavelength, intensity) and/or an external optical, thermal, electrical, acoustic, or mechanical signal.

In the present disclosure, the term optical source refers to a coherent, partially coherent, or incoherent light source which may be based on any technology such as, but not restricted to, light-emitting diodes (LEDs), edge-emitting semiconductor laser diodes, vertical-cavity surface-emitting lasers (VCSELs), MEMS-VCSEL laser, Fourier-domain mode-locked (FDML) laser, white light, and halogen lamps. The wavelength of the light source can be in deep-iltraviolet (DUV), ultraviolet (UV), visible, near-infrared, mid-infrared, or far-infrared ranges depending on the application of the catheter (for example, for imaging, or therapeutic applications). The light may be delivered as pulses of energy (e.g., pulse laser) or as a continuous wave (CW).

Throughout the present disclosure, the term color filter refers to a device that selectively transmits or reflects light of different colors (i.e. wavelengths). Color filters can be based on various mechanisms such as absorption (using a dye, pigment, plasmonic particles, metallic nanostructures), interference (e.g., thin-film, subwavelength grating, Mie resonance structure, plasmonic and metallic nanostructure), or diffraction (e.g., reflective or transmission grating). In this disclosure, a partial mirror may refer to a device that partially reflects incident light. The reflectivity of the partial mirror may be a function of light wavelength and/or its angle of incidence.

Throughout the present disclosure, the imaging sensor may refer to any imaging and sensing technologies to detect or capture light intensity or other light properties such as phase, angle, and wavelength. Some examples of such imaging and sensing technologies include charge-coupled device (CCD), intensified charge-coupled device (ICCD), complementary-symmetry metal-oxide-semiconductor (CMOS), scientific CMOS (sCMOS), avalanche diode (AD), Avalanche-Photodiode (APD), time-of-flight (ToF), Schottky diodes or any other light or electromagnetic sensing mechanism operating at deep-UV, visible, near-infrared, far-infrared and other wavelengths.

Throughout the present disclosure, the ring resonator may refer to a looped optical waveguide where light from an adjacent optical waveguide can couple into the looped optical waveguide. This coupling mechanism is based on interference phenomena and thus it is wavelength dependent. Light coupled into the ring resonator can be coupled out to another waveguide rendering the ring resonator as one kind of wavelength-dependent coupler. The resonance wavelength of the ring resonator (i.e., the wavelength of light that can be coupled into the ring resonator) is determined by design parameters (e.g., material, size, geometries) and it also can be tuned by an external signal.

In this disclosure, Array Waveguide Grating (AWG) may refer to one kind of wavelength division multiplexed system. AWG is capable of de-multiplexing many wavelengths from a single waveguide to multiple waveguides or vice versa (multiplex several wavelengths from different waveguides into a single waveguide).

In this disclosure, Mach-Zehnder may refer to a device that splits up an input light into its two waveguide interferometer arms. Depending on the phase of each arm, light from each arm at their output intersection will experience constructive or destructive interference thus will only be allowed to propagate to a selected output waveguide arm. The phase difference of each waveguide arm can be controlled by an external signal (for example by injecting carriers or heating one (or two) of the interferometric waveguide arms) so one can control the direction of light propagation after Mach-Zehnder.

Further, the present disclosure describes a hybrid approach based on the photonic integrated circuits (PIC), diffractive optics, metasurface, and other flat optical technology (e.g., color filter, polarizer, waveplate, quarter waveplate, a half-wave plate, mirror, partial mirror). Dynamic capability of optical systems may come from electrooptic (by injecting carrier) or thermo-optic effect (by local heating) of PIC components (e.g., ring resonator, Mach-Zehnder), acousto-optic, or other mechanisms and devices such as Liquid Crystal (LC). The dynamic capability significantly enhances the performance and flexibility of optical systems; besides, the multifunctional nature of cascaded planar components enables such systems to satisfy small form factors necessary for medical application. The main focus of the present disclosure is on enabling small form factor, reconfigurable, high-performance optical systems for medical imaging, and therapeutic purposes.

### Miniaturized Imaging and Illuminating System

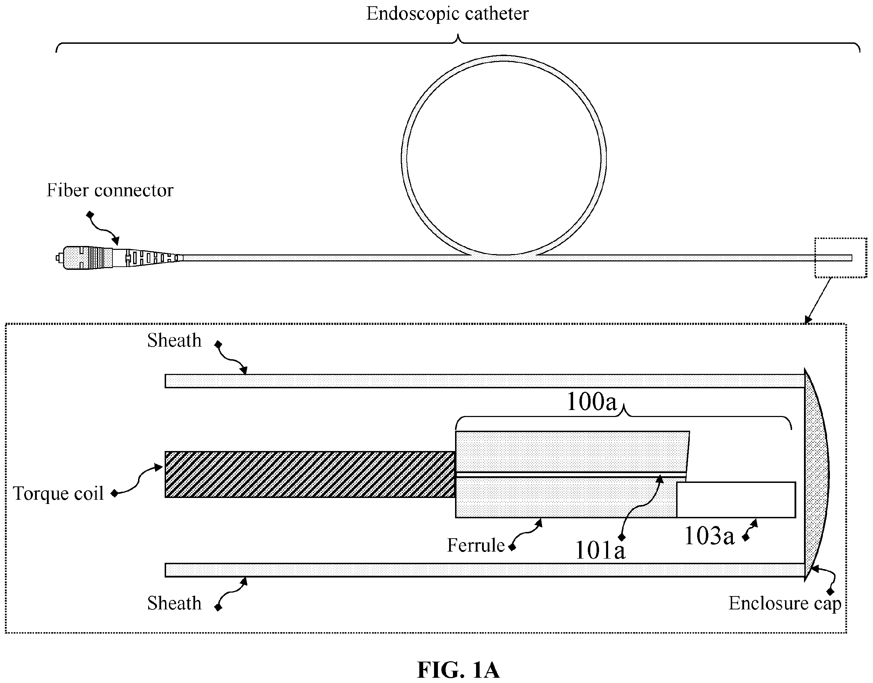

**FIGs. 1A-C** are schematics of two embodiments of miniaturized endoscopic fiber-based catheters.

**FIG. 1A** shows a schematic of the endoscopic catheter including fiber connector, optical fiber, torque coil (to transfer torque from one end of the catheter to the other), and other optical and mechanical components at the distal end. Zoom in view at the bottom of **FIG. 1A** shows the details of components used at the distal end of the catheter including torque coil, optical fiber 101a, which passes through the middle of the torque coil. As shown in **FIG. 1A** (top), at the left side the optical fiber is connected to a fiber connector (such as SC connector, LC connector, etc.) and at the right side to a ferrule. Substrate 103a acts as a platform to hold different optical components used to manipulate and control the light delivered by fiber for imaging and illuminating purposes.

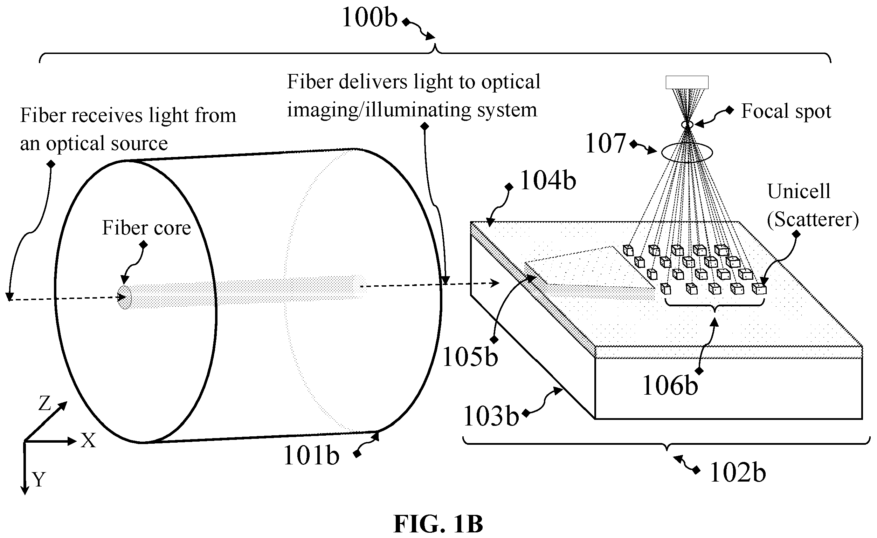

**FIG. 1B** shows the perspective view of an embodiment of a miniaturized endoscopic catheter 100b (for simplicity we only show the distal end of the endoscopic catheter). In this embodiment, the fiber 101b receives light from an optical source (not shown here) and delivers it to the optical imaging and illuminating system (OIIS) 102b that includes a substrate 103b, slab waveguide 104b, tapered waveguide 105b, and Photonic Integrated Circuit based Diffractive Lens (PICDL) 106b. For optical coherence tomography, the operating wavelength may be in the near-infrared (NIR) region (e.g., wavelengths between 800 nm to 1500 nm), which allows for penetration of light into the tissue for depth imaging. Therefore, the OIIS can be built based on Silicon on an Insulator (SOI) platform where silicon, which may include the slab waveguide 104b, has a high refractive index for waveguiding purposes and negligible loss at NIR wavelengths. Due to the size of a silicon waveguide (hundreds of nanometers to a few micrometers in length and/or width) and its high refractive index (for example, n=~3.5), its optical mode is different from that of the fiber. Typical core diameters of single-mode fibers 101b are generally in the range of a several micrometers and have a low refractive index (for example, n= ~1.5).

To increase the coupling efficiency between the fiber and the silicon waveguide, a mode matching mechanism can be used. The mode matching mechanism may include a tapered waveguide 105b or a stack of optical lenses (not shown here) between fiber 101b and the silicon waveguide 104b. Here, tapered waveguide 105b also can be used to expand the optical mode which is coupled in from the fiber 101b to feed the PICDL 106b. PICDL 106b is an array of scatterers (or resonators) on the slab waveguide 104b. These scatterers out-couple the light (propagating in the slab waveguide 104b) toward the focal spot. By design of the size, shape, and location of each scatterer, one can control the amplitude and phase of outcoupled light and thus achieve diffraction-limited focusing with high focusing efficiency. Focusing efficiency is defined as the power of focused light divided by the power of input light (light coupled from fiber).

Notably, the same OIIS 102b can be utilized for illumination (e.g., therapeutic purposes) where one might need to use different wavelengths of light, for example, light in the ultraviolet or visible wavelength range. In that case, other material platforms such as titanium dioxide or hafnium dioxide (with negligible absorption loss in these wavelength ranges and relatively high refractive index of ~2.5) on a glass substrate may be used instead of the SOI platform.

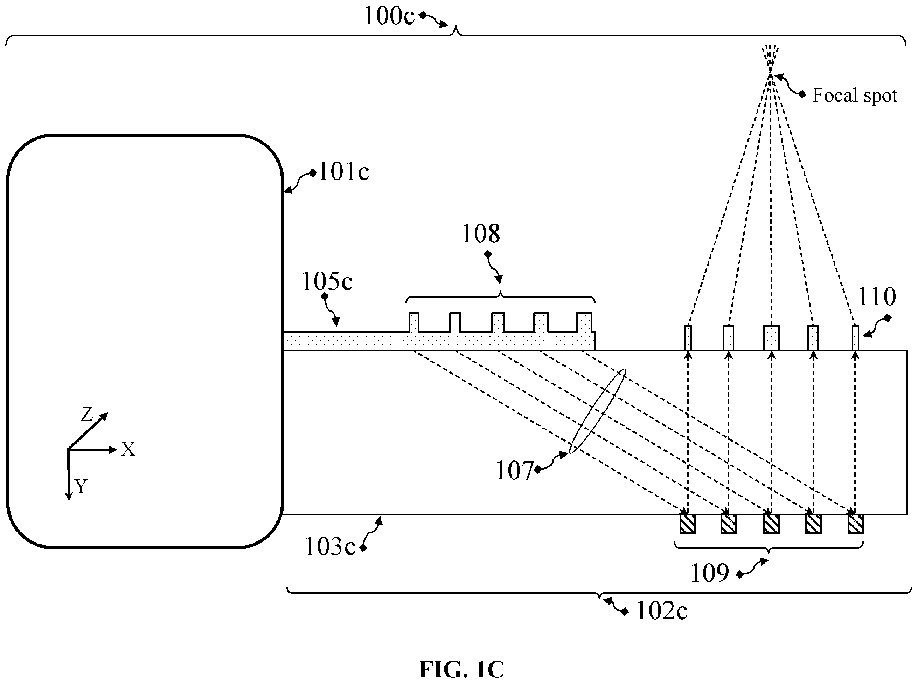

**FIG. 1C** shows the side-view of the second embodiment of a system 100c including a miniaturized OIIS 102c. The system 100c includes an optical fiber 101c, a tapered waveguide 105c, a Photonics Integrated Circuit based Grating (PICG) 108 which may be disposed on or otherwise integrated with the tapered waveguide 105c, a substrate 103c, a grating 109, and a diffractive lens (DL) 110. Here, tapered waveguide 105c facilitates the optical coupling from fiber 101c to the waveguide 105c, and then PICG 108 couples the light into the substrate 103c. Here, the substrate also acts as a guiding medium in which light from the PICG 108 may propagate. Light propagating 107 inside the substate is then diffracted by the grating 109 toward the DL 110 and is focused at the focal spot. Grating 109 and DL 110 may include features that are disposed on or otherwise integrated with one or more sides of the substrate 103. In the embodiment shown, grating 109 may be disposed on a first side of the substrate while DL 110 is disposed on a second side of the substrate that is opposite the first side. One of skill in the art will appreciate that other arrangements are also possible without departing from the scope of the present application.

For both embodiments shown in **FIGs. 1B** **and** **1C****,** we use flat optical components with a height less than a few microns, and considering the substrate is tens of micrometers or hundreds of micrometers thick, we significantly miniaturize the OIIS 102b, 102c along the vertical direction (i.e., the Y-direction in **FIGs. 1B** **and** **1C**)**.** In the radial direction (e.g., not along the X-direction) the size of OIIS 102c is limited by the diameter of DL 110 and other diffractive optical components which can be a few hundreds of micrometers. Thus, embodiments disclosed herein enable an OIIS with a sub-millimeter radial size (along the radius of the fiber). An OIIS of this scale is very hard to achieve with other devices based on prism and angel polished ball lens. By utilizing lenses designed based on metasurface or advanced diffractive optics, one can achieve diffraction-limited imaging in these small diameter lenses, which is hard to achieve by refractive counterparts. This sub-millimeter size OIIS opens a lot of applications such as imaging coronary artery or airways in the peripheral of the lung which is very difficult to do with other available endoscopic catheters due to size limitations.

We can couple the light to the substrate with an angle larger than total internal reflection (TIR). Therefore, light can be guided through the substrate with negligible loss and can be expanded to the desired width before reaching the lens, thereby filling the lens aperture for diffraction-limited focusing. Notably, substrate size along the axial direction (i.e., along the length of fiber) is not limited by human physiology in the case of luminal organ imaging. Expanding the beam's width using other methods, such as by using prisms, comes at the cost of increasing the whole OIIS system size, including along the radial direction.

So far, we explain that the embodiments shown in **FIGs. 1B** **and** **1C** can be used for focusing light into the object (e.g., organs or tissue). Based on the reciprocity principle in optics, the same embodiments (i.e., systems 100b, 100c) can also collect the light scattered by the object and thus form images. In the next section, we will discuss how can we enhance the functionality of these miniaturized OIIS.

### Multi-spectral and Multi-focal Imaging and Illuminating System

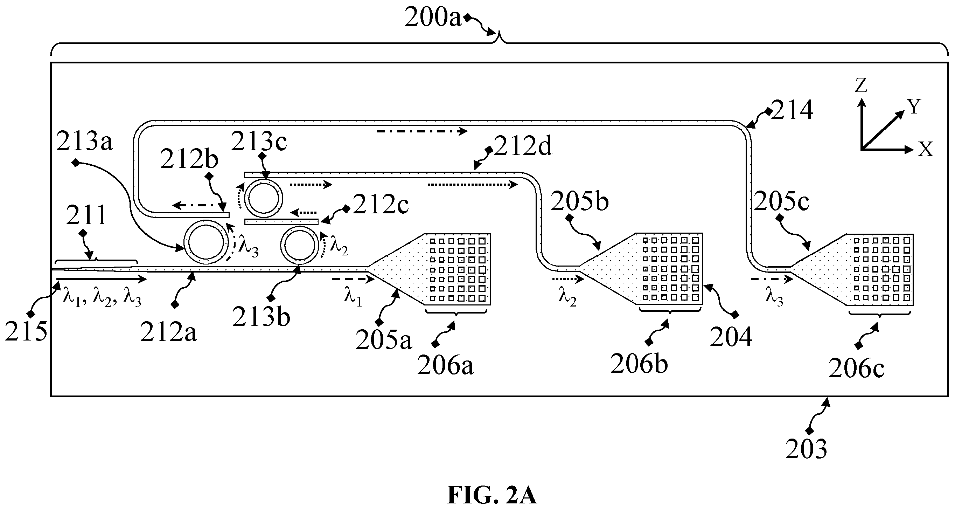

**FIGs. 2A-C** show multi-spectral and multi-focal imaging and illuminating embodiments using Photonic Integrated Circuit (PIC)-based lenses.

The internal diameter of the luminal organ (to be imaged or illuminated) sets a limit on the size of OIIS along the radial direction (e.g., Z and Y-direction in **FIG. 2A**). However, this size limitation is more relaxed along the axial direction (X-direction in **FIG. 2A**). In this section, we discuss how one can cascade PIC components along this X-direction to add functionalities to OIISs which are otherwise very hard to achieve with conventional methods while maintaining a small radial dimension.

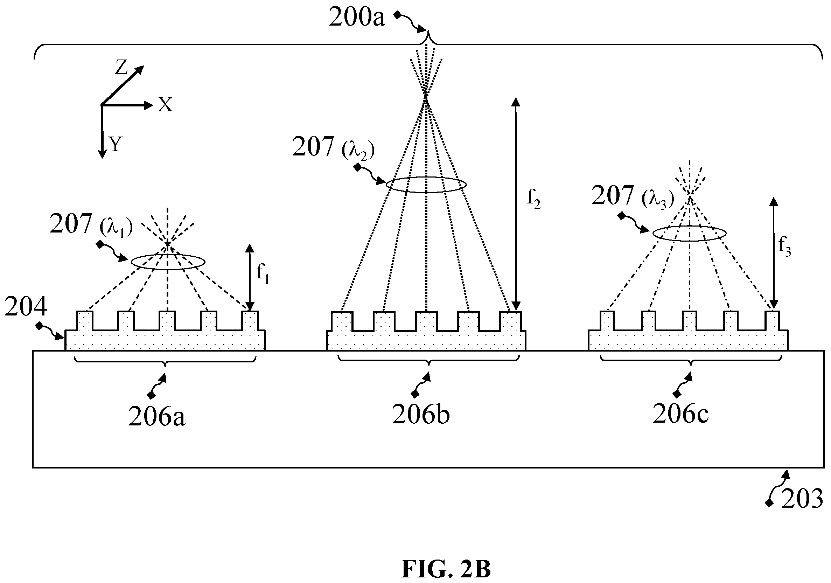

**FIGs. 2A-B** show the top-view (**FIG. 2A**) and side-view (**FIG. 2B**) schematic of multi-spectral and multi-focal OIIS. The tapered waveguide 211 acts as a mode matching medium between the fiber (not shown here) and the first ridge waveguide 212a, thereby enabling efficient coupling of the light from the fiber to the PIC platform 200a. Light 215 coupled from a fiber includes three spectral regions centered at λ₁, λ₂ and λ₃ with a bandwidth of Δλ. Ring resonators 213a-c are used to spectrally sort this multi-spectral input. For example, the ring resonator 213a is designed at wavelength λ₃ so that the portion of input light centered at this wavelength will be coupled into the ring resonator 213a and thus will be re-routed to the second ridge waveguide 212b above it. This light travels toward the waveguide bend 214 and eventually reaches PICDL 206c where it will be outcoupled from the PIC platform 200a and will be focused at a focal length f₃, as determined by the particular design of the array of features within PICDL 206c.

After interaction with 213a, the input light contains light with two spectral ranges centered at λ₁ and λ₂. The ring resonator 213b is designed at λ₂, therefore light centered at this wavelength will be coupled to it from the first ridge waveguide 212a. Next, this λ₂ light in the 213b will be coupled into the the ridge waveguide 212c located on top of 213b and then to ring resonator 213c which is also designed to incouple light at λ₂. Light from 113c will be coupled to the ridge waveguide 212d where it will be directed to PICDL 206b. This λ₂ light will be outcoupled from the PIC platform 200a and will be focused by the PICDL 206b at focal length f₂. The focal length f₂ may be the same or different from the focal length f₃. The remaining light traveling through the first ridge waveguide 212a reaches PICDL 206a and only contains spectral range centered at λ₁ (λ₂ and λ₃ are already coupled to 213a and 213b, respectively) and will be focused at focal length f₁. The focal length f₁ may be the same or different from one or more of the focal lengths f₂ and f₃, and each of the focal lengths f₁, f₂, f₃ are determined by the particular designs of feature arrays within the associated PICDL that outcouples each spectral range of input light.

One of skill in the art will appreciate that although **FIG. 2A** describes dividing input light into three spectral ranges and including three corresponding PICDL components, more or fewer spectral ranges and/or PICDL components could be included within the PIC platform 200a without departing from the scope of the present application. Furthermore, alternate arrangements including more or fewer ring resonators and/or ridge waveguides may be used depending on a particular size, space, orientation, and minimum waveguide path radius constraints on waveguides within the system. As discussed above, the radial dimension may be more important to miniaturize than the axial direction. As such, it may be advantageous to arrange the PICDL components substantially along the less constrained axial direction (i.e., the X-direction) so as to reduce the size in the radial direction (e.g., in the Z-direction and Y-direction). Scatterers of PICDL may have different shapes (i.e., cylindrical, pillar with rectangular, square, hexagonal, or arbitrarily cross-sections) diameter, width, length, or height. Each scatterer can be a single nanostructure of an arbitrary shape and/or array of at least two nanostructures.

This platform 200a is exceptional from several perspectives. First, we sort each wavelength of light to the desired PICDL specifically designed at that wavelength. This ensures high focusing efficiency and diffraction-limited imaging resolution (it is well known that both metasurface and diffractive-based lens's performance degrades when operating at a wavelength different from the design wavelength) at each spectral range.

Second, using this platform 200a, we can arbitrarily adjust the focal length of an OIIS by changing the wavelength of input light thus achieving a multi-focal imaging system as illustrated in the side view drawing of PIC platform 200a (**FIG. 2B**). In other words, using this multi-focal OIIS, we can increase the lateral imaging resolution in the desired plane (along the Y direction, normal to axial-direction) located at focal length distance from the PICDL where the light is tightly focused. In the context of medical imaging, this means we can increase the lateral imaging resolution at the desired depth which is adjustable by the wavelength. Also, by stitching multiple focal lengths (each focal spot will have a depth of focus) close to each other, we can effectively extend the depth of focus (depth of field) of OIIS beyond a normal optical system. By cascading and repeating this architecture, we can increase the number of spectral regions and thus focal lengths. For example, a luminal organ may have different internal diameters along its length, therefore the ability to adjust the focal length is important to be able to perform imaging at different locations of the organ without a need to exchange the imaging systems. Further, the wavelength for imaging and illumination (for therapeutic purposes) may be different. The ability to operate at a different wavelength within the same embodiment enables diagnosis (via imaging) and treatment (via light illumination and exposure) within the same embodiment. Depending on these operating wavelengths, one may need to use different materials in the same platform to assure high efficiency and performance at each wavelength (for example silicon at near-infrared and titanium dioxide at the visible range).

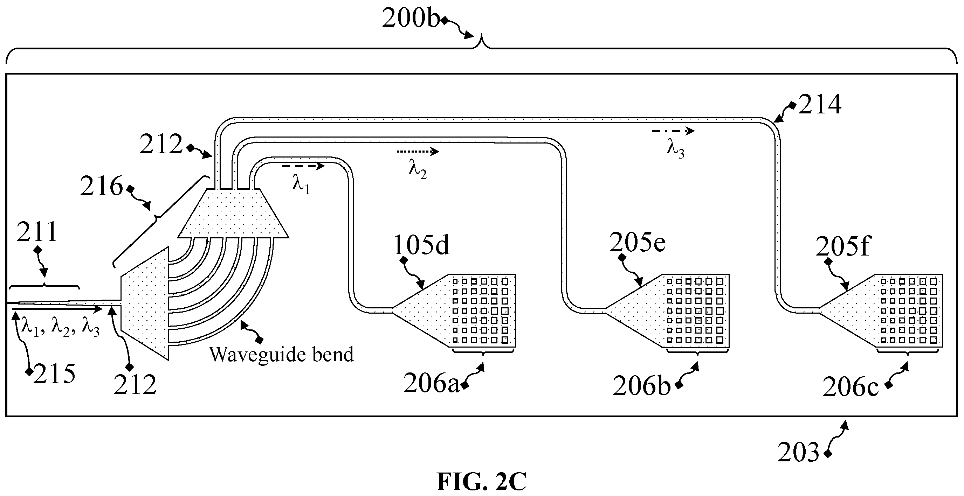

Redirecting light based on its center wavelength can be done utilizing other PIC components than ring resonators, such as array waveguide grating (AWG) as shown in PIC platform 200b illustrated in **FIG. 2C****.** Here, AWG 216 includes two trapezoid shape parts connected by six waveguide bends. Depending on the wavelength of light, AWG re-directs the light toward the desired PICLD (λ₁ to 206a, λ₂ to 206b, λ₃ to 206c) enabling a multi-spectral and multi-focal optical system. Here, for exemplary purposes, we consider three PICDLs, but there is no limit to how many PICDLs one can cascade to increase the number of focal lengths and spectral bandwidth.

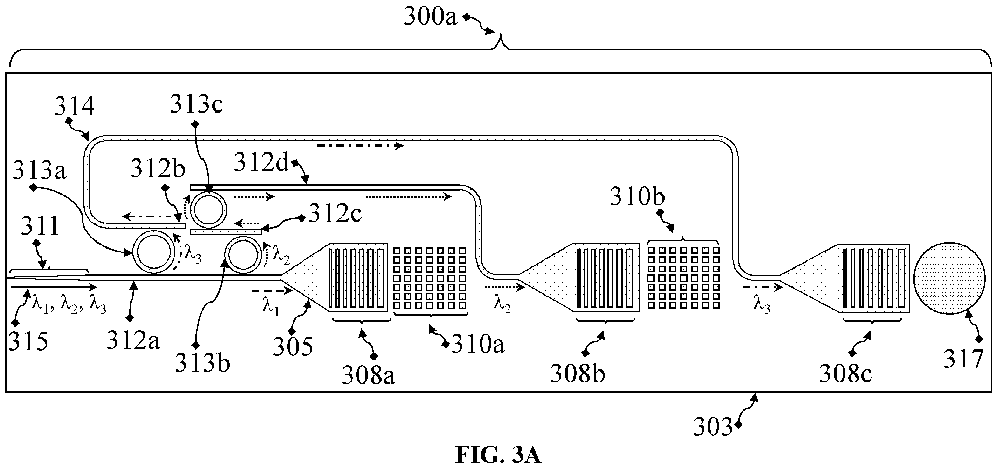

**FIGs. 3A-B** show a multi-spectral and multi-focal imaging embodiment using Photonic Integrated Circuit (PIC)-based gratings.

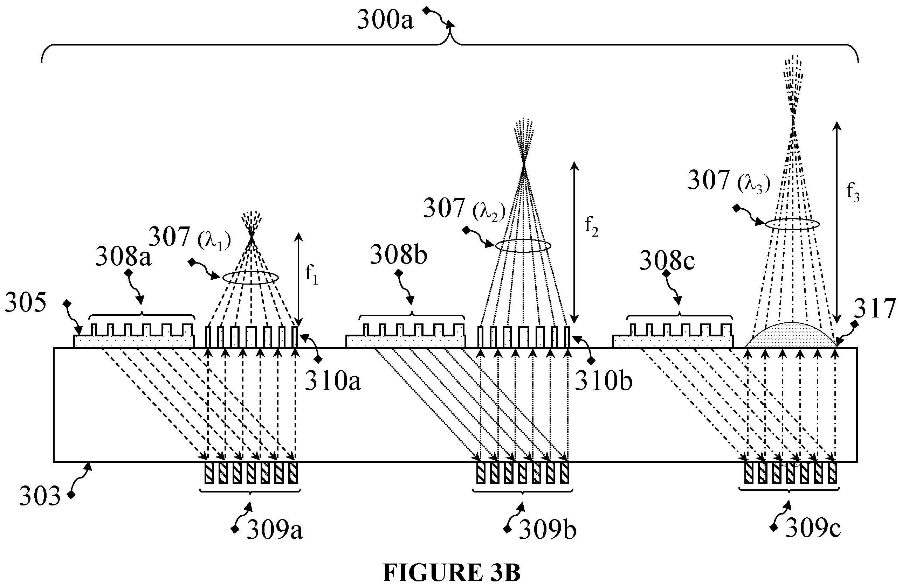

**FIG. 3A** shows the top-view of the multi-spectral and multi-focal embodiment of a PIC system 300a. Three-ring resonators (313a, 313b, and 313c) sort the input light based on its center wavelength and re-direct them to the desired PICG. For example, light with the center wavelength λ₃ will be coupled into the 313a and then coupled out from 313a into the ridge waveguide 312b above it. This light eventually will be coupled into the substrate 303 by PICD 308c (as shown in the side view illustration in **FIG. 3B**). Light with the center wavelength λ₂ will be re-routed to the PICG 308b via 313b, waveguide 312c, 313c, and waveguide 312d. Finally, light with the center wavelength λ₁ propagates inside the ridge waveguide 312a without coupling into any of the ring resonators and reaches PICG 308a. As shown in **FIG. 3B****,** PICG 308a, PICG 308b and PICG 308c will couple light into the substrate 303 toward gratings 309a, 309b, and 309c, respectively. For example, the light, coupled into the substrate by 308a, will impinge on the first grating 309a and will be diffracted toward 310a to be focused at focal length f₁. Note that the light center at each wavelength has a spectral range that can be small or large depending on the design and will experience chromatic dispersion upon interaction with PICGs (308a, 308b, 308c). One of the main functions of 309a is to compensate for the chromatic response of 308a and to re-direct the light toward the 310a with the chief ray being normally incident on the DL310a. In other words, 310a is designed to be an on-axis diffractive lens to avoid the lateral chromatic aberration and its associated negative effect (e.g., degrading lateral resolution of the imaging system). Note that the chief ray can have a different angle than 90 degrees (relative to the horizontal line), in that case, 310a is designed in such a way that does not change the angle of the chief ray and focuses the light along the chief ray trajectory. Similarly, light with the center wavelengths λ₂ and λ₃ will be focused at focal lengths of f₂ and f₃, respectively. Here, by cascading three PICGs with other PIC-based components, we realize an imaging platform that can perform multi-spectral (λ₁, λ₂, and λ₃) and multi-focal (f₁, f₂, and f₃) imaging in a small form factor.

Note that in many embodiments, including that illustrated in **FIGs. 3A** and **3B****,** we can use, as a matter of design choice, diffractive and/or refractive lenses (refractive lens 317 can be spherical, aspherical, or freeform optics lens made of glass, silicon, or any other material suitable for operating wavelength) to focus the light. This is another powerful aspect of this embodiment where depending on the requirement, we can change the focusing lens without the need to change the rest of the imaging system (e.g., PIC parts). Here, for simplicity in illustrating the system 300a, we cascaded three of the sub-imaging-system (PICG, grating, and lens), and we can cascade more of these sub-imaging systems if required to increase the number of achievable focal lengths and broaden the spectral range. Having different focal lengths for each of these sub-imaging systems also expands the depth of focus (depth of field) of the OIIS and enhances its lateral resolution at multiple depths.

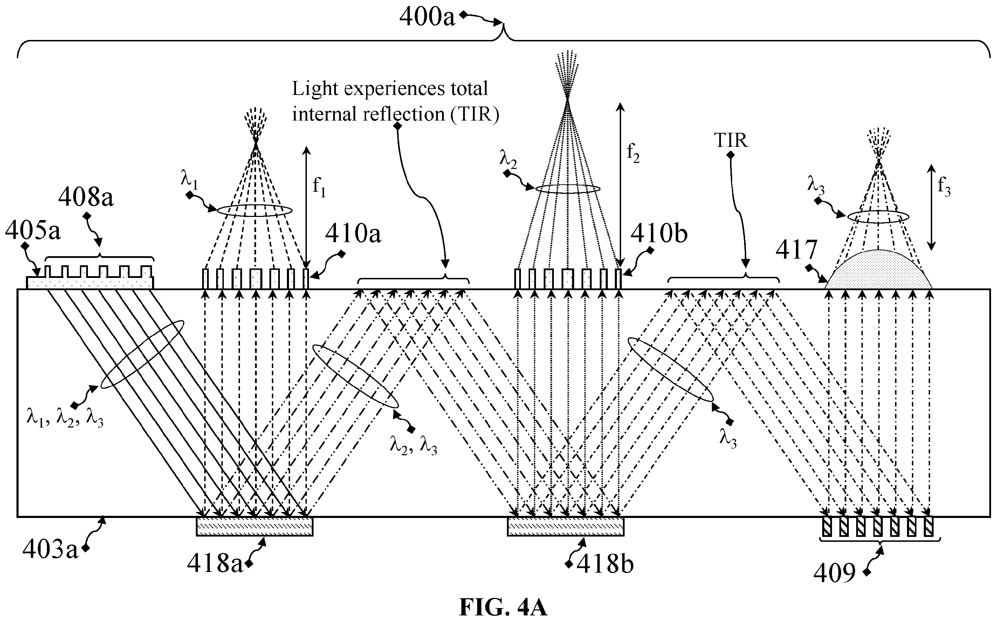

**FIGs. 4A-C** show multi-spectral and multi-focal imaging embodiments based on cascaded gratings.

**FIG. 4A** shows the side-view of a multi-spectral and multi-focal OIIS 400a based on wavelength-selective gratings (WSG) (418a and 418b). First PICG 408a couples multi-spectral light (three wavelengths) into the substrate 403a from a fiber (not shown). Here, we assume the angle of coupled light is larger than the TIR angle of the substrate at all three wavelengths; therefore, light is trapped and propagates inside the substrate 403a. We also assume a special case where three wavelengths (λ₁, λ₂, λ₃) are diffracted to the same angles inside the substrate which can be either achieved using achromatic grating at PICG 408a (e.g., grating-based on metasurface design) or by considering these three wavelengths being very close to each other. In this embodiment, we utilize WSGs (418a, 418b) to re-route the light based on its wavelength. Light, coupled into the substrate by 408a, first interacts with WSG 418a, which may be disposed on a side of the substrate opposite the side on which the PICG 408a is disposed. Designed at λ₁, 418a diffracts this wavelength λ₁ and does not affect (i.e., does not diffract) wavelengths λ₂ and λ₃. Thus, light centered at the wavelength λ₁ is re-directed toward the DL 410a and will be focused at the focal length f₁. DL 410a may be disposed on the same side of the substrate as PICG 408a. Light with the wavelength of λ₂ and λ₃ continue to propagate inside the substrate 403a and, after a TIR interaction from the top surface of the substrate, λ₂ will interact with and will be diffracted by WSG 418b which is designed to act on light at wavelength λ₂. Light at wavelength λ₂ will eventually interact with and will be focused by the DL 410b at the focal length f₂. Light centered at the wavelength of λ₃ will not be affected by WSG 418b and will propagate toward the grating 409 after two TIR interactions. Grating 409 may be different from 418a and 418b in that it may not be a wavelength selective grating; rather, grating 409 may act on all wavelengths of light that reach it. In this case, the only remaining wavelength of light to reach the grating 409 is λ₃ and so wavelength selectivity may not be required. After being diffracted by the grating, this λ₃ light will be focused by the refractive lens 417 at the focal length f₃. Here, by cascading three gratings (two WSGs and one normal grating), we focus light with different wavelengths at the different focal lengths. This optical system 400a is a reciprocal system meaning the same system will be used to collect the light scattered from the object (e.g., tissues or organs) and sent it back toward the fiber which delivers it to the OCT system (not shown) for post-processing and image forming.

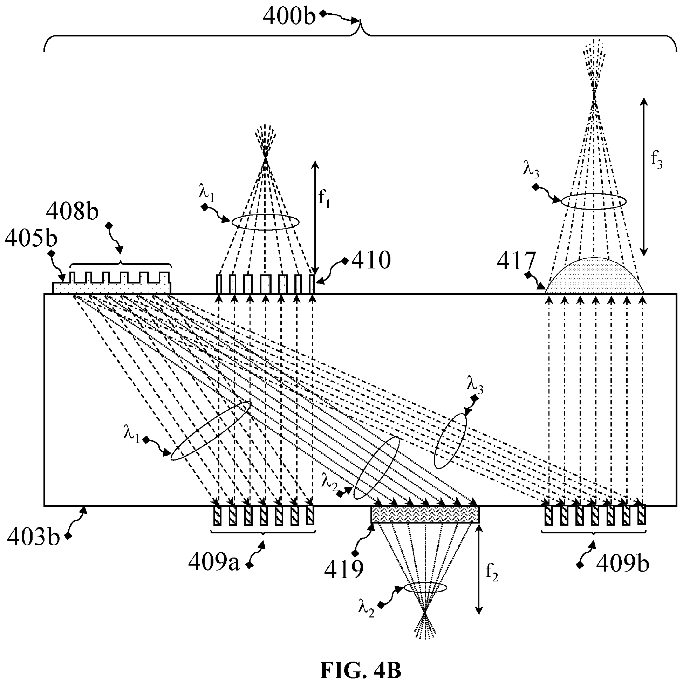

**FIG. 4B** shows the side-view of an OIIS 400b which represents another embodiment for the case where wavelength difference between coupled lights is substantial and PICG 408b acts as normal grating (e.g., wherein diffracted angle is a function of wavelength). Therefore, the PICG 408b couples and spatially separates light of different wavelengths inside the substrate 403b. Light centered at λ₁ will be diffracted by grating 409a and then focused by DL 410 at focal length f₁ as the light λ₁ outcouples from the substrate 403b. Light with wavelength λ₂ impinges on a diffractive element 419. This diffractive element 419 is a superposition of a diffractive lens and diffractive grating. In other words, it simultaneously couples the light out of TIR and focuses it at focal length f₂. Notably, this wavelength λ₂ light may outcouple in a different direction than light associated with wavelength λ₁. Other embodiments are possible wherein wavelength λ₂ light is relayed such that it outcouples from the substrate along a same direction as the wavelength λ₁ light, and this may be a matter of design choice. Finally, light with wavelength λ₃ will be diffracted by grating 409b toward the refractive lens 417 and will be focused at focal length f₃. While a refractive lens 417 is shown, a diffractive lens similar to DL 410 may be interchanged as a matter of design choice. Here in OIIS 400b, we utilize the intrinsic dispersive response of the PICG 408b to realize an OIIS 400b with multi-focal functionality which can also perform imaging at several spectral ranges (multi-spectral imaging).

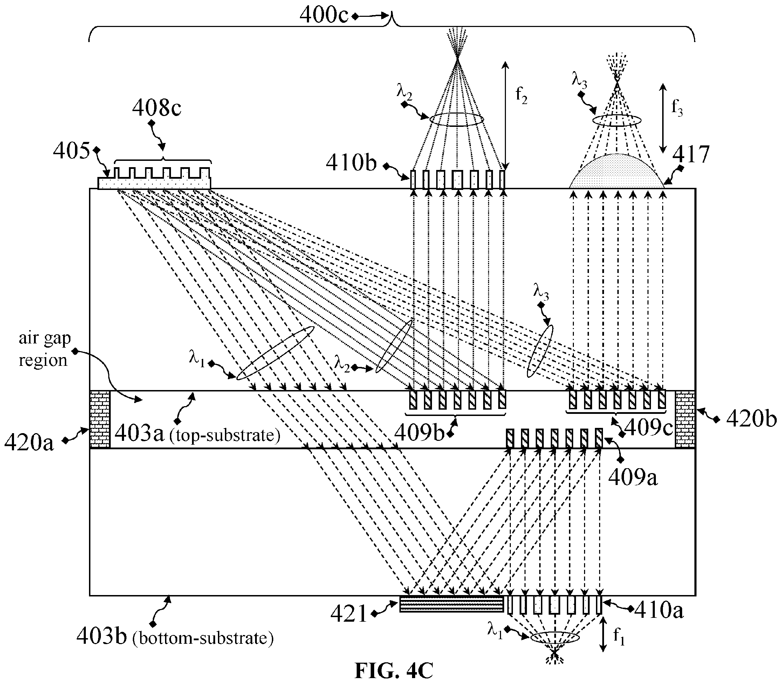

In some cases, it might be possible that the angle of the light coupled by the PICG 408c into the substrate is smaller than that of the TIR angle of the substrates (403a and 403b). This case is shown OIIS 400c in **FIG. 4C** where the light with wavelength λ₁ is not coupled into the TIR mode thus exits from the bottom surface of the top-substrate 403a and enters the air gap region (e.g., a gap between top-substrate and bottom-substrate which are separated by spacers 420a and 420b) and then enters the bottom-substrate 403b. Here, we assume the top-substrate 403a and bottom-substrate 403b are made of the same material therefore λ₁ light is not bound by TIR in the bottom-substrate either. To redirect this λ₁ light toward grating 409a, we use a mirror 421 (e.g., metallic or dielectric). Next, the grating 409a, which may be disposed on the bottom substrate 403b within the air gap, diffracts this λ₁ light toward the DL 410a to be focused at focal length f₁. DL 410a may be disposed on an outer surface of the bottom substrate 403b so as to outcouple λ₁ light from the bottom substrate 403b. Light centered at wavelengths λ₂ and λ₃ will be focused at focal length f₂ and f₃, respectively after interaction with corresponding gratings and lenses (λ₂ with grating 409b and DL 410b, λ₃ with grating 409c and refractive lens 417). In the OIIS 400c, 409b and 409c are both disposed on top-substrate 403a within the air gap and both DL 410b and refractive lens 417 are disposed on the outer surface of top-substrate 403a so as to outcouple light from the top substrate 403a. Here, we consider an embodiment with two substrates separated by spacers 420a and 420b creating an air gap between substrates. We can vertically stack more substrates with different optical components to enhance the capability (e.g., number of focal lengths and bandwidth of multi-spectral imaging) of this embodiment.

### Reconfigurable Multi-Focal Imaging System

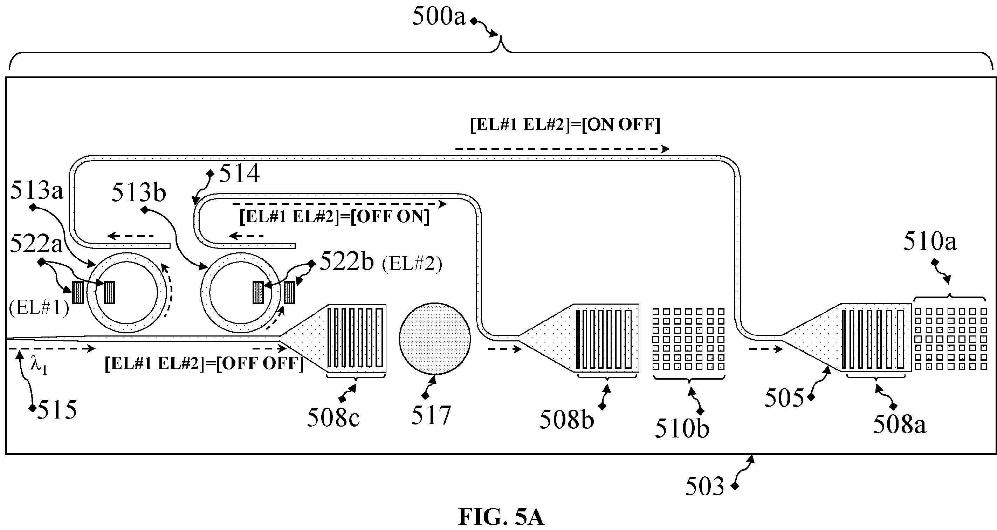

**FIGs. 5A-C** show three different embodiments of reconfigurable multi-focal imaging and illuminating systems.

FIG. 5A shows the top-view of a multi-focal OIIS 500a with three adjustable focal lengths (f₁, f₂, and f₃). Here, ring resonators (513a and 513b) are used to dynamically re-route the light to selected lenses with the desired focal length. By injecting carrier or generating heat via electrodes 522a (EL#1) and 522b (EL#2), one can modify the refractive index of the medium near the ring resonators thus changing their resonance wavelength (i.e., the wavelength of light that is coupled into the ring resonator). Therefore, one can selectively and dynamically re-route the input light to one of the lenses (refractive lens 517, DL 510a and DL 510b) and consequently adjust the focal length of the OIIS 500a. When both electrodes (EL#1 and EL#2) are "OFF" the ring resonators (513a and 513b) are off-resonance (i.e., are not able to incouple light at wavelength λ₁). Therefore the input light 515 which has a wavelength λ₁ will not couple into either of the ring resonators and will reach PICG 508c and then the refractive lens 517 with the focal length f₃. Turning "ON" the EL#1 switches the resonance wavelength of the first ring resonator 513a to λ₁ (i.e., the wavelength of input light). Thus the input light λ₁ is coupled into 513a and re-directed to PICG 508a via a ridge waveguide and eventually to DL 510a to be focused at focal length f₁. If we turn "OFF" the EL#1 and turn "ON" the EL#2, the second ring resonator 513b will be on-resonance at λ₁ so that the input λ₁ light will be coupled into it and re-directed toward PICG 508b via a ridge waveguide and eventually will be focused at focal length f₂ by DL 510b. We can readily increase the number of the adjustable focal length of OIIS 500a by cascading this embodiment using additional PICGs, and associated ring resonators, waveguides, and lenses.

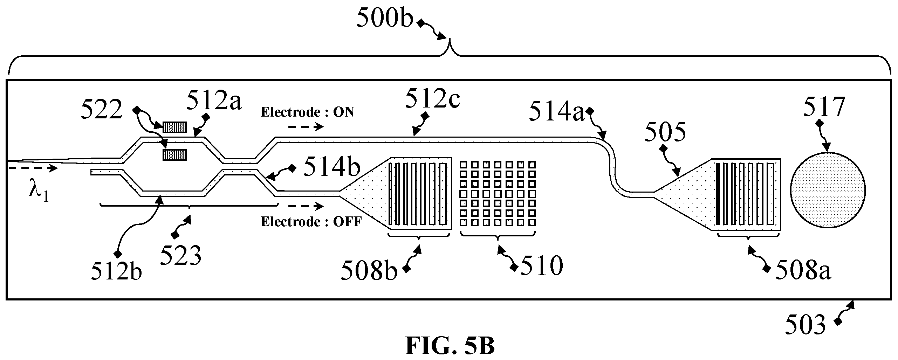

**FIG. 5B** shows the top-view of the second embodiment of a multi-focal OIIS 500b using Mach-Zehnder modulator 523. By switching the electrodes 522 between "ON" and "OFF" states, we can control the phase shift that input light experiences passing through the top arm (i.e., the ridge waveguide 512a at the middle of two electrodes 522) of the Mach-Zehnder. The phase control mechanism can be based on either an injecting of carriers or a heating mechanism via electrodes. Both carrier injection and local heating can change the refractive index of the medium surrounding the top arm (e.g., a ridge waveguide 512a) of the Mach-Zehnder thus one can control the phase shift that input light experiences when passing through the top arm 512a. With electrodes 522 in the "ON" state, input light λ₁ continues propagating in the top arm of the Mach-Zehnder and reaches the PICG 508a via 512c and eventually will be focused by the refractive lens 517 at focal length f₁. With electrodes 522 in the "OFF" state, the light will be re-routed to the bottom arm 514b (e.g., at the end of Mach-Zehnder) and reaches PICG 508b. This light will be eventually focused at focal length f₂ by DL 510. Here, we achieve an OIIS 500b with a reconfigurable focal length via an electric signal selectively delivered to electrodes on the OIIS 500b. One can increase the number of achievable focal lengths by cascading more Mach-Zehnders which re-route the input light to more PICGs and other components (e.g., gratings, lenses) to focus at different focal lengths.

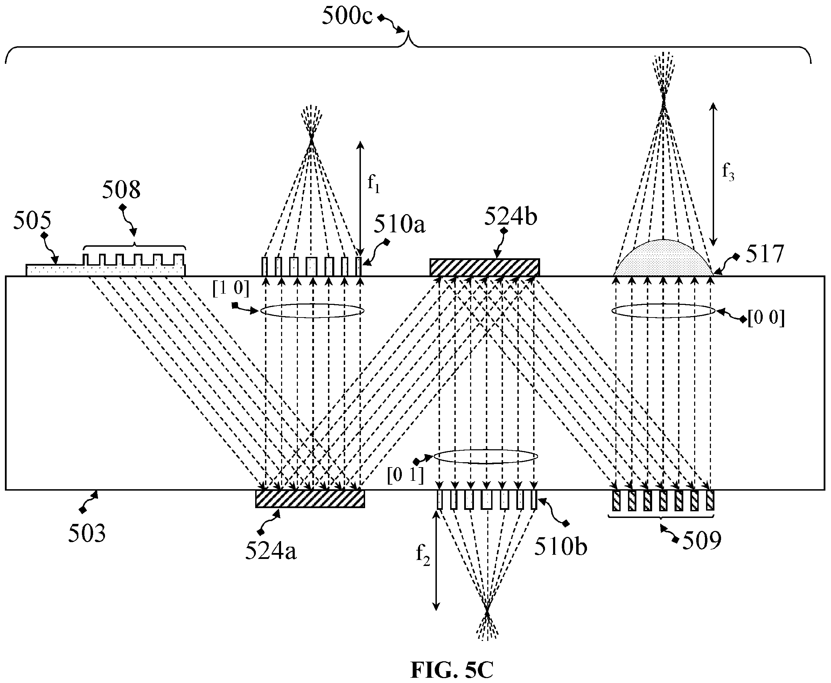

**FIG. 5C** shows the side-view of the third embodiment of a multi-focal OIIS 500c based on switchable Liquid Crystal Gratings (LCG). After coupling into the substrate 503 by the PICG 508, input light comprising wavelength λ₁ interacts with first LCG 524a. In the "ON" state, the LCG 524a acts as grating and diffracts the light toward the DL 510a, and the DL 510a focuses the light at focal length f₁. Here, we assume the second LCG 524b is in the "OFF" state (i.e., [1 0]). When LCG 524a is "OFF", and LCG 524b is "ON" (i.e., [0 1]), the input light is not be affected (i.e., diffracted) by LCG 524a and continues propagating in TIR mode until reaches LCG 524b. The input light is then diffracted by the LCG 524b toward the DL 510band eventually is focused at the focal length f₂. The last case is when both LCG 524a and LCG 524b are "OFF" ([0 0]), so the input light is not affected by 524a and 524b and reaches the grating 509. Grating 509 diffracts light toward the refractive lens 517 and which is then focused at focal length f₃. Here, we show that by switching LCGs, we can adjust the focal length of the OIIS 500c: in [0 0], [0 1], and [1 0] states, the OIIS will have associated focal lengths of f₃, f₂, and f₁, respectively. We may increase the number of achievable focal lengths by cascading LCGs and adding more gratings, lenses, and other appropriate components.

### Polarization Resolved Imaging and Illuminating System

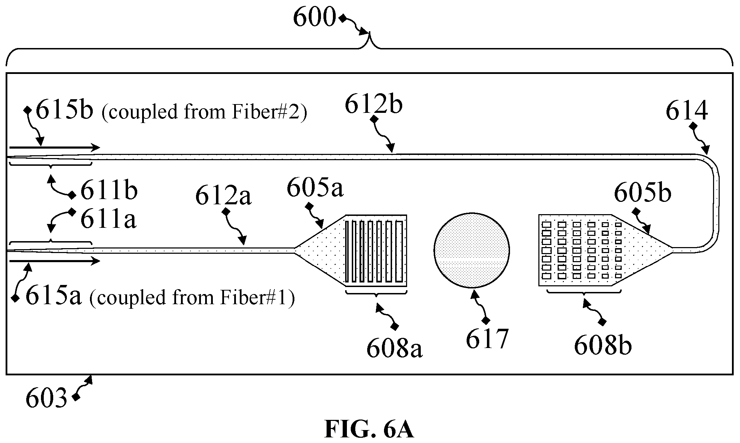

**FIGs. 6A-B** show an embodiment of a miniaturized polarization-resolved imaging and illuminating system.

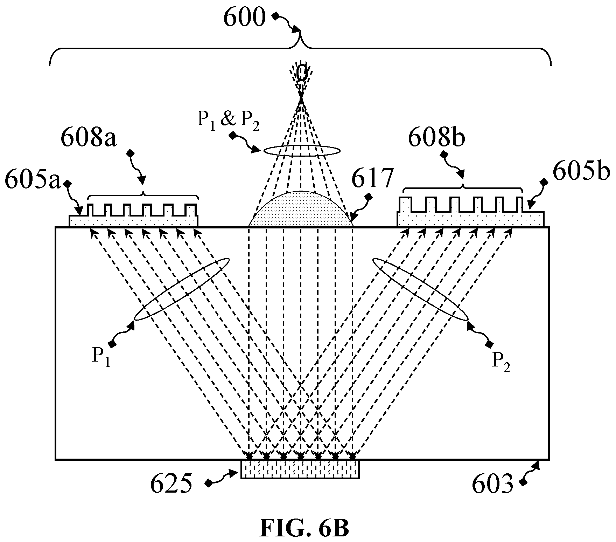

**FIG. 6A** shows the top-view of OIIS 600 which is a polarization-resolved imaging and illuminating embodiment. Here, we assume two fibers (or a single dual-core fiber) with each fiber (or core) connected to one of the PICG (608a or 608b) via tapered waveguide (611a or 611b) and ridge waveguide (612a or 612b). For example, a first fiber or first fiber core may be coupled with PICG 608a, and a second fiber or second fiber core may be coupled with PICG 608b. Input light traveling through the first and second fibers (or first and second fiber cores) may contain one or both polarization states P₁ and P₂ of light. We also assume PICGs are designed to be polarization-dependent: PICG 608a only couples P₁ polarization and PICG 608b only couples P₂ polarization into the substrate 603 (where P₁ and P₂ are two orthogonal states of polarization, either linear, circular, elliptical, or any combination of those). Then, as shown in the side-view of this embodiment (**FIG. 6B**), the polarization dependent grating (PDG), 625 will diffract the P₁ and P₂ light toward the refractive lens 617. Here, the refractive lens acts as a polarization-independent lens (focus both polarization P₁ and P₂ at the same focal spot). Furthermore, we can achieve a polarization-independent lens using a diffractive lens or metasurface-based lenses instead of the refractive lens 617.

Having a reciprocal system here, as shown in **FIG. 6B****,** the refractive lens 617 may also collect light scattered by the object regardless of its polarization and sends it to the PDG 625. Depending on the polarization of the light, the PDG 625 diffracts the light toward PICG 608a or PICG 608b (i.e., P₁ to 608a, P₂ to 608b). Eventually, the collected P₁ and P₂ light is coupled to first and second fibers (or fiber cores), respectively, and is sent back to the OCT system (not shown) for polarization-resolved image processing. Here, we just discuss one embodiment for exemplary purpose, and there are several alternative designs to achieve polarization-resolved imaging using PIC-based components and diffractive optics. For example, we can use a binary grating instead of PDG, which results in losing 50% of light for illumination and 50% of light for collection (assuming PICGs only collect one polarization of the light). We can also integrate this platform with other PIC-based components such as ring resonators or Mach-Zehnders to add more functionality such as multi-focal and multi-spectral imaging. One can also cascade the embodiment shown in **FIGs. 6A-6B** to achieve several polarization-resolved imaging sub-systems on the same substrate with different focal lengths, numerical apertures, and working distances and switch between them by external electrical signal utilizing Mach-Zehnder or other PIC-based components as discussed in other embodiments herein.

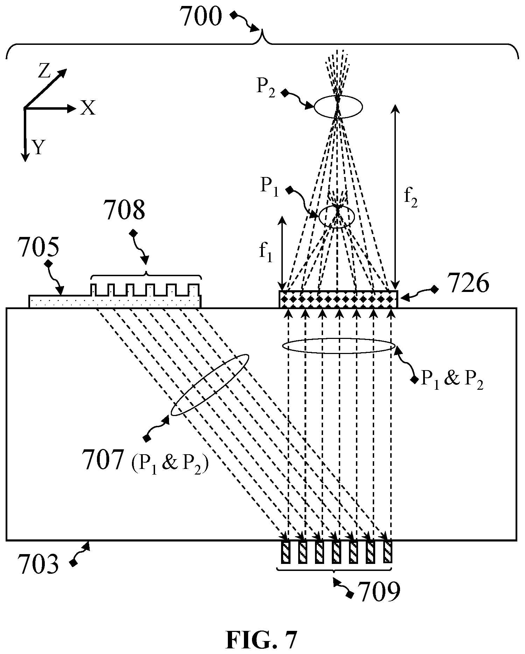

**FIG. 7** shows an embodiment of a fiber-based catheter imaging and illuminating system with an extended depth of focus.

**FIG. 7** shows the side-view of the embodiment of OIIS 700 to extend the depth of focus (depth of field) utilizing a polarization-dependent lens (PDL) 726. In this embodiment, PICG 708 couples input light (regardless of its polarization) into the substrate 703 toward a grating 709. Then, the coupled light (i.e., light containing P₁ and P₂ polarization where P₁ and P₂ are two orthogonal states of polarization) is diffracted by the grating 709 toward the PDL 726. PDL 726 is designed in such a way that focuses P₁ polarization at the first focal length f₁ and P₂ polarization at the second focal length f₂ thus extending the depth of focus of the OIIS 700. In other words, like any other lens, each focal spot of PDL has a depth of focus, we can design these focal lengths f₁ and f₂ in such a way that their depth of focus overlaps. Therefore, we can extend the depth of focus of the OIIS starting before f₁ and ending after f₂. Since in OCT imaging the lateral resolution (i.e., resolution in the X-direction and Z-direction) at each imaging plane (i.e., planes orthogonal to the transverse Y-direction of the fiber) is defined by how tightly light is focused, extending the depth of focus enhances the imaging resolution thus improving 3D imaging resolution and contrast.

### Compact Form Factor Beam Expander

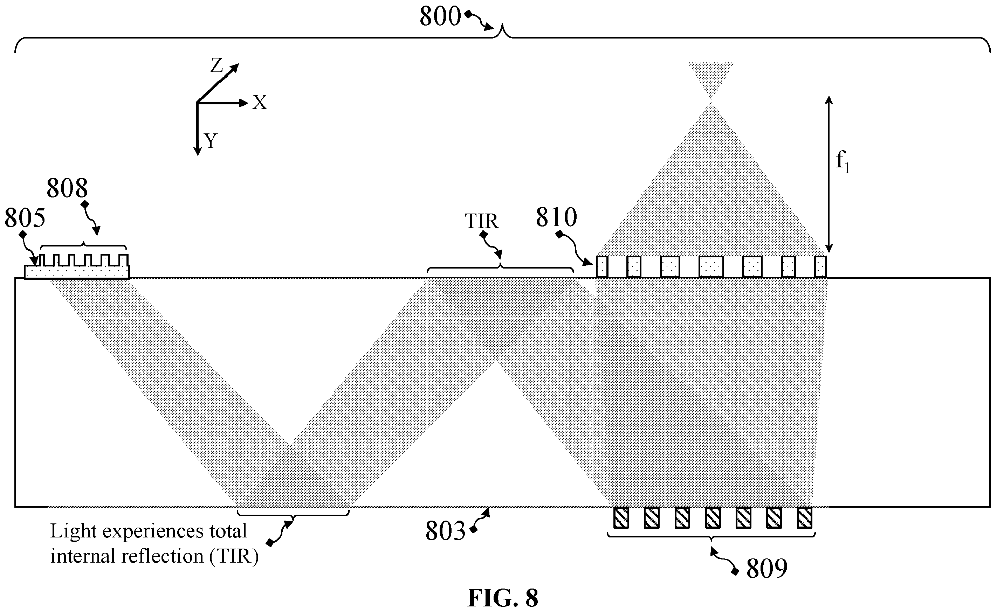

**FIG. 8** shows a compact form factor embodiment to expand the beam's width.

One of the powerful aspects of the platform that we disclose here is it may be used to expand the input light without a need to add any extra components or increase the size of any existing components along the radial direction (e.g., top to bottom of the page). This concept is shown in **FIG. 8** in OIIS 800 where light is coupled to the TIR mode of the substrate 803 by the PICG 808. This light can propagate inside the substrate with a negligible loss. Owing to the substate's relatively large thickness (thickness of the substrate can be smaller or larger than tens of micrometers, smaller or larger than hundreds of micrometers, and smaller or larger than a few millimeters depending on design requirements) and width (thickness and width are defined along the Y-direction and Z-direction, respectively) compared to the wavelength of light, we can treat the substrate as free space with a refractive index n (i.e., the refractive index of substrate material). Therefore, light naturally expands (diverges) while propagating inside the substrate (see **FIG. 8**). We can control the beam's width by adjusting its propagation length that the beam travels in such a way that the beam's width is equal to the diameter of the lens used for focusing when the beam interacts with the lens. This beam expansion mechanism also allows having a smaller PICG 808 compared to the lens 810 and thus reducing the nano-fabrication cost and time. In other words, PICG's aperture does not need to be as large as the lens diameter since we can expand the beam by propagating it.

### Extra Functionality with Vertical Stacking

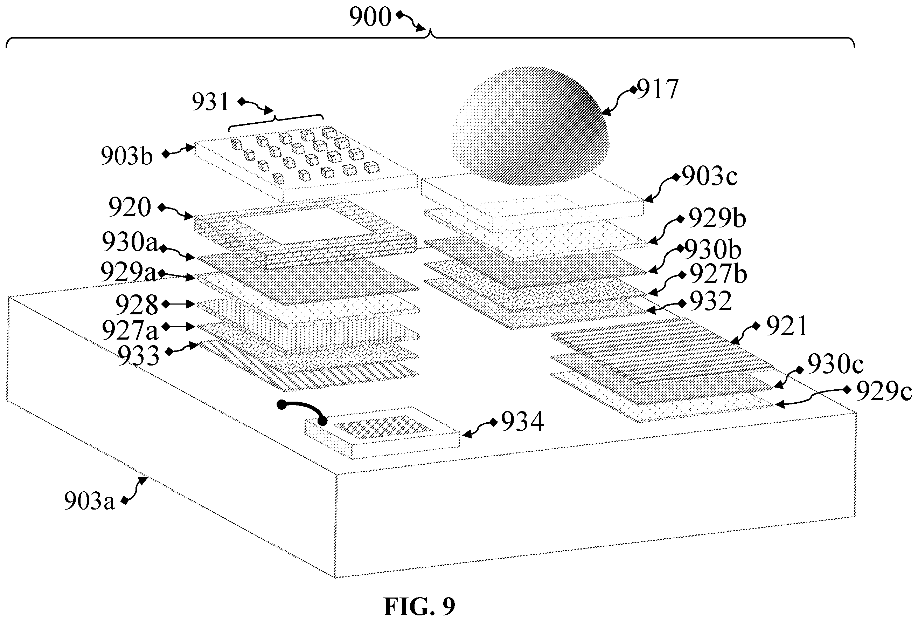

**FIG. 9** is an exploded view of an embodiment of an optical imaging and illuminating system.

Most of the components we use in the proposed embodiments discussed herein are planar which can be easily vertically stacked with other planar components such as mirrors 921, color filters 927, partial reflectors 928, waveplate 929, polarizers 930, holograms 931, thin-film 932 (e.g., anti-reflection (AR) coating), and liquid crystals 933. This vertical integration capability can exceptionally expand the functionality of the imaging/illuminating system described here. For example, by stacking liquid crystals 933 and waveplates 929 (e.g., half-waveplate and quarter-waveplates) one may control/change the polarization of light at will or remove unwanted polarization. Some other examples are stacking thin film 932 and a color filter 927 to control the reflection or transmission of light depending on its wavelength. We also can use thin-film to make an antireflection coating on the substrate surface to avoid reflection loss. We also consider integrating a sensor/detector 934 on the OIIS platform which can get feedback from the imaging scene. One example of a sensor is a depth sensor to measure the distance of the tissue (object) from the lens to accordingly adjust the lens's focal length.

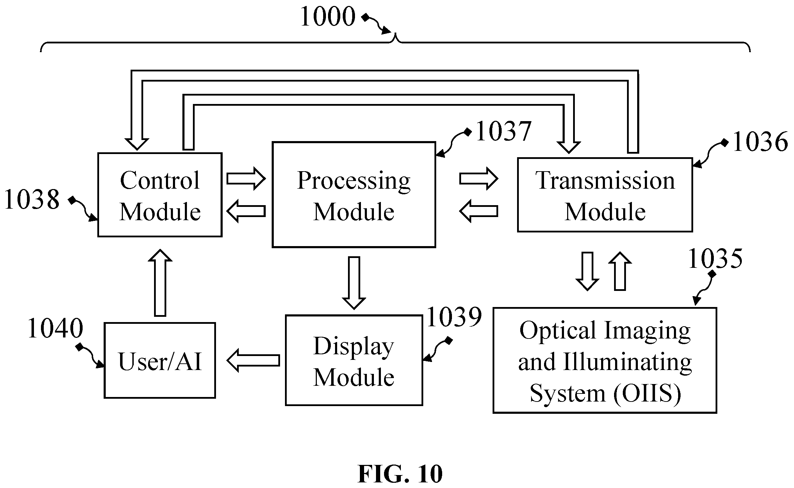

**FIG. 10** is a block diagram of different modules for implementing the methods disclosed herein, in accordance with some embodiments.

**FIG. 10** shows a high-level schematic 1000 of different modules and systems and how they work together to improve the performance of the whole imaging system that may include one or more OIIS embodiments as discussed previously herein. The OIIS 1035 is designed to focus light into the object and then collect scatter light from the object to form an image. The OIIS 1035 receives the input light from the Processing Module 1037 via the Transmission Module 1036. The Transmission Module at least includes a single-mode fiber and/or at least a multimode fiber. There may be a case where transmission is done wirelessly and may not need any fiber. Fibers transmit the input light from a source (e.g., laser, LED) to the OIIS and then collect the image information from the OIIS back to the Processing Module 1037. Furthermore, the Transmission Module may include at least one electrical wire and/or at least one wireless transmitter. Electrical wire and/or the wireless transmitter can be used to transmit an electrical or electromagnetic signal between the sensor 1034 (not shown here, may be part of 1035) and the Processing Module. Processing Module may include at least one interferometric arm (in case of optical coherence tomography imaging) for image processing purposes, at least one photodetector, at least one camera, at least one imaging sensor, at least one a photodetector, and/or at least one spectrometer. All of these components in the Processing Module will be collectively used to form and analyze images and send them to the Display Module 1039. User/AI (Artificial Intelligence) Module 1040 receives image information from the Display Module and then decides which parameters in the Processing Module or OIIS need to be changed/adjusted to improve the image quality. User/AI 1040 makes require changes and adjustments via the Control Module, Processing Module, and Transmission Module.

The foregoing description and figures are illustrative of various embodiments of the present invention and are not to be construed as limiting thereof. Although a few exemplary embodiments of this invention have been specifically described, those skilled in the art will readily appreciate that many modifications are possible in the exemplary embodiments without materially departing from the novel teachings and advantages of this invention as defined by the appended claims. Accordingly, many different embodiments stem from the above description and the drawings.

## Claims

1. An optical system (102b) for an endoscope comprising:
a substrate (103b) having a flat surface;
an input aperture configured to receive light from an optical fiber (101b) in the endoscope, the received light propagating along an axial direction of the substrate;
an optical routing structure comprising a flat optical component supported by the flat surface of the substrate;
a focusing component, wherein the optical routing structure routes light from the input aperture to the focusing component and also redirects the light from an axial direction to a transverse direction, and the focusing component focuses the routed light to a focal spot located to a side of the optical system;
**characterized in that** the system further comprises
a lens (106b) selected from a group consisting of a photonic integrated circuit (PIC) lens, a diffractive lens and a meta-lens, wherein the lens is supported by the flat surface of the substrate, and the lens functions as both the focusing component and as the flat optical component of the optical routing structure.

2. The optical system of claim 1 wherein all of the optical components in the optical routing structure are flat optical components.

3. The optical system of claim 1 wherein the light travels by total internal reflection through the substrate.

4. The optical system of claim 1 wherein the optical routing structure does not contain a prism or turning mirror.

5. The optical system of claim 1 wherein, within the optical routing structure, the light is redirected only by PIC, diffractive and/or meta optical components.

6. The optical system of claim 1 wherein a maximum transverse dimension of the optical system does not exceed 3 mm, and/or the flat optical component has a thickness of not more than 200 µm.

7. The optical system of claim 1 wherein the focusing component has an extended depth of focus.

8. The optical system of claim 1 further comprising:
a slab waveguide (104b) supported by the substrate, wherein the light from the optical fiber is coupled through the input aperture into the slab waveguide.

9. The optical system of claim 8 wherein the slab waveguide includes a tapered section for mode matching between the optical fiber and the slab waveguide.

10. The optical system of claim 8 wherein the slab waveguide is a silicon on insulator (SOI) waveguide.

11. The optical system of claim 1 wherein the light has a wavelength in a range of 800-1500 nm.

12. The optical system of claim 1 further comprising:
a vertical stack of at least two flat optical components, wherein the light propagates out of the substrate through the vertical stack to the focal spot.

13. The optical system of claim 12 wherein the vertical stack includes at least two of: a color filter, a partial reflector, a waveplate, a polarizer, a hologram, a thin-film, and a liquid crystal layer.

14. An endoscope system comprising:
an insertion tube having a distal tip;
an optical fiber running along the insertion tube; and
any of the optical systems of any of the preceding claims, the optical system located at the distal tip and receiving light from the optical fiber.

15. The endoscope system of claim 13 further comprising at least one of:
a torque coil that rotates the optical system around the axial direction of the substrate; and
a processing module; wherein light scattered by the focal spot(s) is collected by the optical system and the processing module produces images from the collected light using optical coherence tomography imaging principle.

## Patentansprüche

1. Optisches System (102b) für ein Endoskop, umfassend:
ein Substrat (103b) mit einer flachen Oberfläche;
eine Eingangsöffnung, die konfiguriert ist, um Licht von einer optischen Faser (101b) im Endoskop zu empfangen, wobei sich das empfangene Licht entlang einer axialen Richtung des Substrats ausbreitet;
eine optische Leitstruktur, die eine flache optische Komponente umfasst, die von der flachen Oberfläche des Substrats getragen ist;
eine Fokussierungskomponente, wobei die optische Leitstruktur Licht von der Eingangsöffnung zur Fokussierungskomponente leitet und das Licht außerdem von einer axialen Richtung in eine transversale Richtung umlenkt, und die Fokussierungskomponente das geleitete Licht auf einen Brennpunkt fokussiert, der sich an einer Seite des optischen Systems befindet;
**dadurch gekennzeichnet, dass** das System ferner eine Linse (106b) umfasst, die aus einer Gruppe ausgewählt ist, die aus einer Photonik-IC-Linse (PIC-Linse), einer diffraktiven Linse und einer Metalinse besteht, wobei die Linse von der flachen Oberfläche des Substrats getragen ist und die Linse sowohl als Fokussierkomponente als auch als flache optische Komponente der optischen Leitstruktur fungiert.

2. Optisches System nach Anspruch 1, bei dem alle optischen Komponenten in der optischen Leitstruktur flache optische Komponenten sind.

3. Optisches System nach Anspruch 1, bei dem sich das Licht im Substrat durch Totalreflexion ausbreitet.

4. Optisches System nach Anspruch 1, bei dem die optische Leitstruktur weder ein Prisma noch einen Umlenkspiegel enthält.

5. Optisches System nach Anspruch 1, bei dem das Licht innerhalb der optischen Leitstruktur nur durch PIC-, diffraktive und/oder metaoptische Komponenten umgelenkt wird.

6. Optisches System nach Anspruch 1, bei dem die maximale Querabmessung des optischen Systems 3 mm nicht überschreitet und/oder die flache optische Komponente eine Dicke von nicht mehr als 200 µm aufweist.

7. Optisches System nach Anspruch 1, bei dem die Fokussierungskomponente eine erweiterte Fokustiefe aufweist.

8. Optisches System nach Anspruch 1, das ferner umfasst:
einen vom Substrat getragenen Plattenwellenleiter (104b), wobei das Licht aus der optischen Faser durch die Eingangsöffnung in den Plattenwellenleiter gekoppelt wird.

9. Optisches System nach Anspruch 8, bei dem der Plattenwellenleiter einen sich verjüngenden Abschnitt für die Modenanpassung zwischen der optischen Faser und dem Plattenwellenleiter umfasst.

10. Optisches System nach Anspruch 8, bei dem der Plattenwellenleiter ein Silizium-auf-Isolator-Wellenleiter (SOI-Wellenleiter) ist.

11. Optisches System nach Anspruch 1, bei dem das Licht eine Wellenlänge im Bereich von 800 bis 1500 nm aufweist.

12. Optisches System nach Anspruch 1, das ferner umfasst:
einen vertikalen Stapel aus mindestens zwei flachen optischen Komponenten, wobei sich das Licht aus dem Substrat durch den vertikalen Stapel zum Brennpunkt ausbreitet.

13. Optisches System nach Anspruch 12, bei dem der vertikale Stapel mindestens zwei von einem Farbfilter, einem Teilreflektor, einer Wellenplatte, einem Polarisator, einem Hologramm, einer Dünnschicht und einer Flüssigkristallschicht umfasst.

14. Endoskop-System, umfassend:
ein Einführrohr mit einer distalen Spitze;
eine entlang des Einführrohrs verlaufende optische Faser; und
ein beliebiges der optischen Systeme aus einem der vorstehenden Ansprüche, wobei sich das optische System an der distalen Spitze befindet und Licht von der optischen Faser empfängt.

15. Endoskop-System nach Anspruch 13, das ferner mindestens eines von
einer Drehmomentspule, die das optische System um die Achsenrichtung des Substrats dreht; und
einem Verarbeitungsmodul umfasst; wobei das von dem/den Brennpunkt(en) gestreute Licht vom optischen System gesammelt wird und das Verarbeitungsmodul Bilder aus dem gesammelten Licht unter Verwendung des Bildgebungsprinzips der optischen Kohärenztomographie erzeugt.

## Revendications

1. Système optique (102b) pour un endoscope comportant :
un substrat (103b) ayant une surface plane ;
une ouverture d'entrée configurée pour recevoir de la lumière provenant d'une fibre optique (101b) dans l'endoscope, la lumière reçue se propageant le long d'une direction axiale du substrat ;
une structure d'acheminement optique comportant un composant optique plat supporté par la surface plane du substrat ;
un composant de focalisation, dans lequel la structure d'acheminement optique achemine de la lumière de l'ouverture d'entrée vers le composant de focalisation et redirige également la lumière depuis une direction axiale vers une direction transversale, et le composant de focalisation focalise la lumière acheminée vers un point focal situé sur un côté du système optique ;
**caractérisé en ce que** le système comporte en outre une lentille (106b) sélectionnée dans un groupe constitué d'une lentille à circuit intégré photonique (PIC), d'une lentille diffractive et d'une méta-lentille, dans laquelle la lentille est supportée par la surface plane du substrat, et la lentille fonctionne à la fois comme composant de focalisation et comme composant optique plat de la structure d'acheminement optique.

2. Système optique selon la revendication 1, dans lequel tous les composants optiques dans la structure d'acheminement optique sont des composants optiques plats.

3. Système optique selon la revendication 1, dans lequel la lumière circule par réflexion interne totale à travers le substrat.

4. Système optique selon la revendication 1, dans lequel la structure d'acheminement optique ne contient pas de prisme ou de miroir tournant.

5. Système optique selon la revendication 1, dans lequel, au sein de la structure d'acheminement optique, la lumière est redirigée uniquement par des composants PIC, diffractifs et/ou méta-optiques.

6. Système optique selon la revendication 1, dans lequel une dimension transversale maximale du système optique ne dépasse pas 3 mm, et/ou le composant optique plat présente une épaisseur n'excédant pas 200 µm.

7. Système optique selon la revendication 1, dans lequel le composant de focalisation présente une profondeur de focalisation étendue.

8. Système optique selon la revendication 1 comportant en outre :
un guide d'ondes en plaque (104b) supporté par le substrat, dans lequel la lumière provenant de la fibre optique est couplée à travers l'ouverture d'entrée dans le guide d'ondes en plaque.

9. Système optique selon la revendication 8, dans lequel le guide d'ondes en plaque comporte une section effilée pour la correspondance de mode entre la fibre optique et le guide d'ondes en plaque.

10. Système optique selon la revendication 8, dans lequel le guide d'ondes en plaque est un guide d'ondes en silicium sur isolant (SOI).

11. Système optique selon la revendication 1, dans lequel la lumière présente une longueur d'onde dans une plage de 800 à 1500 nm.

12. Système optique selon la revendication 1 comportant en outre :
un empilement vertical d'au moins deux composants optiques plats, dans lequel la lumière se propage hors du substrat à travers l'empilement vertical jusqu'au point focal.

13. Système optique selon la revendication 12, dans lequel l'empilement vertical comporte au moins deux éléments parmi : un filtre de couleur, un réflecteur partiel, une lame d'onde, un polariseur, un hologramme, un film mince et une couche de cristaux liquides.

14. Système d'endoscope comportant :
un tube d'insertion présentant une extrémité distale ;
une fibre optique s'étendant le long du tube d'insertion ; et
l'un quelconque des systèmes optiques selon l'une quelconque des revendications précédentes, le système optique étant situé à l'extrémité distale et recevant de la lumière en provenance de la fibre optique.

15. Système d'endoscope selon la revendication 13, comportant en outre au moins l'un parmi :
une bobine de couple qui fait tourner le système optique autour de la direction axiale du substrat ; et
un module de traitement ; dans lequel la lumière diffusée par le point focal ou les points focaux est collectée par le système optique et le module de traitement produit des images à partir de la lumière collectée à l'aide du principe d'imagerie par tomographie par cohérence optique.
